# EUROPEAN PATENT SPECIFICATION

(11) **EP 1 789 793 B1**
(45) Date of publication and mention of the grant of the patent: **19.10.2011**
(21) Application number: 05777646.0
(22) Date of filing: 08.07.2005
(51) Int. Cl.: G01N 33/558

(54) **COMBINATION ASSAY FOR ALCOHOL AND DRUGS OF ABUSE**
KOMBINATIONSTEST FÜR ALKOHOL UND DROGEN
ESSAI COMBINÉ POUR DÉTECTION D'ALCOOL ET DE DROGUES

(30) Priority: 09.07.2004 US 888029
(43) Date of publication of application: 30.05.2007
(60) Divisional application: 11167050.1
(73) Proprietor: Branan Medical Corp., Irvine, CA 92618 (US)
(72) Inventor: WONG, Raphael, Irvine, CA 92618 (US); ZOLTEK, Richard, North Webster, IN 46555 (US)
(74) Representative: von Kreisler Selting Werner
(86) International application number: PCT/US2005/024262
(87) International publication number: WO 2006/017175

(56) References cited:
- WO-A-96/38720
- WO-A-99/22238
- WO-A1-88/08534
- US-A- 4 900 666
- US-A- 5 556 789
- US-A1- 2002 001 818
- US-A1- 2002 045 195
- US-B1- 6 203 757
- US-B1- 6 248 598

## Description

### FIELD OF THE INVENTION

The present invention relates to an apparatus and method to collect bodily fluids and provide an assay of one or more analytes present in those bodily fluids.

### BACKGROUND OF THE INVENTION

Drugs of abuse and alcohol are the most frequent causes of driving under the influence, in addition to a host of other problems related to their use. For example, illegal drug use and excessive use of alcohol contribute to many accidents, injuries and medical conditions. Screening individuals for drugs of abuse and alcohol is an important method in identifying those who may cause harm to themselves and others. Screening may also provide an additional benefit as a deterrent against inappropriate and/or illegal use of drugs or alcohol. To that end, many tests have been developed in order to screen individuals for the presence of drugs of abuse and alcohol, or metabolites or other residue thereof. For example, some such test apparatus and methods involve the determination of the presence and/or amount of drugs of abuse or alcohol in biological fluids, such as blood, urine, and oral fluid. These have proven to be useful analytical methods.

However, the chemical methods of choice for such tests have often included complex laboratory procedures, such as gas chromatography for analyzing blood or urine, and a range of laboratory or field tests. These tests involve time-consuming procedures, and thus are not useful for a rapid determination of the intoxication of an individual, such as might be needed during a roadside traffic stop.

Thus, there is an increasing demand for a simple, accurate, and reproducible method for determining the presence of drugs of abuse or alcohol in bodily fluids. Not only would such a test lead to more rapid determinations of intoxication or sobriety during traffic stops, but also could be used in other fields, such as to assure that operators of dangerous equipment, such as heavy construction equipment or military equipment, are not intoxicated. Additionally, there is an added benefit that the rapid identification of such individuals aids in removing those individuals from the operation of automobiles and other equipment, thereby reducing costs, both in human terms (i.e., injuries, lives lost, etc.) and financial terms.

In order to provide a more rapid analysis, a number of testing devices, using rapidly reacting test strips, have been developed. In general, a sample of bodily fluids such as urine, blood, or oral fluid, is applied to the test strip in order to effect a reaction, such as an immunological or enzymatic reaction, to determine the presence of the analytes being tested. For example, one such test strip for drugs of abuse present in substances such as blood, urine, serum, and tissue, uses immunological principles. In particular, drug-specific antibodies and antigens have been used in a variety of immunological assay procedures for detecting antibodies or antigens in bodily fluids of humans and animals. Test devices are known which can identify the presence or absence of drugs of abuse, such as cocaine, opiates, and marijuana, using the protein conjugates of these drug derivatives and their accompanying antibodies.

In addition to the immunoassay test strips described above, other test strips use enzymatic reactions in order to determine the presence of alcohol in bodily fluids. Such devices are useful due to the prevalent use of alcohol tests in society today. For example, approximately one-third of all patients currently admitted to hospital emergency rooms are tested for blood alcohol levels for the purpose of making a correct judgment as to the nature of the patient's clinical condition. Before the advent of such test strips, measurements were determined by taking blood samples by venipuncture and hand carrying the sample to a laboratory for a blood alcohol determination. The previous procedure had taken from thirty minutes to as long as a few hours.

Newer tests, however, have included test strips employing the enzyme alcohol oxidase. Alcohol oxidase is a particularly unstable enzyme that undergoes rapid deterioration and loss of activity. Particularly, alcohol oxidase reacts with alcohol to form a hydrogen peroxide. The hydrogen peroxide can then be reacted with a chromagen, or other detectable marker, in order to produce the appearance of a particular color on a test strip, signifying the presence of alcohol in the body.

Most on-site drugs of abuse tests are based on lateral flow immunoassay, which is a dynamic assay with an undefined sample size. The enzymatic assay of alcohol is an end point assay with defined sample volume. By nature, these two types of tests require different sample applications and different result interpretations. Examples of commercially available drugs of abuse tests and alcohol tests are: Oratect™ Multiple Drug Screen COC/MET/THC/AMP/OPL/PCP Oral Fluid Test (Branan Medical Corporation, Irvine, California), and Alco-Screen (Chematics, North Webster, Indiana), respectively. Further, U.S. Patent No. 6,248,598 discloses an immunoassay that provides for both collection of oral fluid and an assay of oral fluid for one or more analytes with a visual readout. The '598 patent discloses a device which collects oral fluid and initiates an assay or assays on the oral fluid, but does not disclose a device which may assay for both drugs of abuse and alcohol.

Thus, some drawbacks remain with current test apparatus. For example, while various tests have been developed, both for drugs of abuse and for alcohol, the drugs of abuse tests operate on immunological principles, while the alcohol tests operate via enzymatic reactions. In general, it is difficult to combine these two types of tests into one device. For example, devices which use various test strips (or other membranes) to house components of both drugs of abuse and alcohol tests, experience the disadvantage of reagents for the different tests cross-reacting with one another. For example, the reagents of the alcohol test used to effect color change can migrate to the immunoassay strip to interfere with the test for drugs of abuse. As a result of difficulties such as those described above, separate devices must presently be used when testing for both drugs of abuse and alcohol. This need for separate test strips increases the amount of equipment that must be kept on-hand in order to perform such tests, as well as increasing the time it takes to administer multiple different tests. The costs of keeping several different test devices on hand are increased, as well. Further, any test method that uses samples of blood and/or urine may also be invasive to the individual being tested.

In view of the above, additional assays and methods are desirable.

### SUMMARY OF THE INVENTION

The present invention overcomes the above-described drawbacks of testing apparatus and methods by providing a test apparatus that allows testing for the presence of both drugs of abuse and alcohol within a single device. In general, the apparatus of the present invention includes an immunoassay for qualitative screening for various drugs of abuse and a reactive pad including an enzyme as a test for alcohol. The two types of tests are contained within the same housing and these tests are performed using the same sample source from a subject being tested.

In particular, the present invention provides an apparatus for testing for multiple analytes, which includes a housing contacting a sample collection pad that partially extends from the housing. The collection pad is in wicking contact with a single backing material supporting (either directly or indirectly): (1) a first test strip having components comprising a sample pad and an absorption pad in wicking communication for an immunological assay wherein said sample pad and said absorption pad are each in wicking communication with said first test strip, and (2) a second test strip having components for an enzymatic reaction. In use, sample in the collection pad contacts both first and second test strips for both immunological and enzymatic detection of an analyte or analytes contained in the sample wherein the first and the second test strip are each associated with the collection pad, but not associated with one another. Thus, once a sample is deposited on the collection pad, it flows to both the first and second test strips to trigger both the immunological and enzymatic tests.

The present invention also provides a method for testing for both drugs of abuse and alcohol by providing an apparatus as described above, initiating an immunological assay and an enzymatic reaction, and detecting the results of the immunological assay and the enzymatic reaction. In general, initiating the immunological assay and the enzymatic reaction includes delivering a sample to the collection pad. One then waits a predetermined amount of time for the sample to travel by wicking action to the first and second test strips, and then obtains control and test results on the test strips visually, by an automatic readout device, etc.

The test apparatus and method of the present invention may use oral fluid as the sample. Because oral fluid is used instead of blood, urine, or another bodily fluid as the test sample, the apparatus avoids the problems described above of intruding on privacy, invasiveness to the person, and can also avoid the problem of sample alteration. That is, because the subject may be observed while taking the test, the opportunity for the subject to use a phantom sample not his/her own, or to alter his/her own sample, is eliminated. Further, due to their size, simple and rapid nature, etc., the test strips can be used on job sites, in schools, and can be customized for rapid screening of illegal use of controlled materials or other substances.

The physical and chemical character of the device of the present invention assays both types of analytes within a single test housing. While the assays are run at the same time using the same sample, the readouts may not necessarily appear at the same time, although it will be beneficial for the readouts to appear close in time to one another.

More specifically, the apparatus of the present invention includes a housing having a collection pad for collection of oral fluid. This collection pad further includes an absorbent material that, when placed in the mouth of a subject, provides for absorption of oral fluid to the collection pad. The oral fluid flows along the collection pad via a wicking action (i.e., capillary action) to a first test strip and a second test strip. The use of separate test strips prevents the reagents of the alcohol test from cross-reacting with the drugs of abuse test, and vice-versa. This is because the reagents for each of the tests are not housed on the same strip or membrane. While the first and second test strips are each associated with the collection pad, they are not associated with one another. One test strip is used to test for drugs of abuse and the other test strip is used to test for the presence of alcohol. The designations of "first" and "second" are used for convenience only and need not indicate any reaction order. Either test strip may be designated as "first" or "second."

The apparatus further Includes test reagents on each of the first and second test strips, which are used to detect the presence of any sought-after drug or alcohol analytes. More specifically, the first test strip has components or reagents including a detectable marker specifically adapted to bind a drug analyte to be detected. The marker is adapted to bind analyte by being conjugated to an antibody to the drug analyte being tested. The marker is not immobilized or otherwise bound to the first test strip, and moves along the first test strip with the flow of the sample oral fluid. The first test strip further includes a drug analyte immobilized at a test region on the test strip. This drug analyte corresponds to the drug being tested for in the sample. The marker is located on the first test strip between the point where the collection pad receives the sample and the immobilized drug analyte. If the sample of oral fluid includes any one of the drugs to which antibody and analyte are present, the drug(s) are bound by antibody present in the conjugate pad. Thus, the corresponding antibody will not be available to bind analyte in the test region [detectable by the assay as it flows past the test region]. This is a positive result. If there is no drug analyte in sample, then the antibody present in the conjugate pad will be free to bind drug analyte in the test region. The antibody analyte conjugate in the test region will create a detectable signal, due to the detectable marker, indicating a test that is negative for the presence of drug analyte.

The second test strip includes enzyme reagents specifically adapted to effect a color change of a particular portion of the test strip, the reactive pad, when alcohol is present in the oral fluid sample. In general, the enzymatic test for alcohol includes an enzymatic pad, including alcohol oxidase and peroxidase. This enzymatic pad is in communication with a wicking membrane that is, in tum, operatively connected to the collection pad. The reactive pad, on contact with solutions of alcohol, will rapidly turn different colors, in one embodiment from shades of green to blue, depending on the amount of alcohol present.

In another embodiment, the present invention also provides a method for detecting analytes in the oral fluid of a subject. The method includes providing an apparatus generally as described above, collecting oral fluid on the first portion of the apparatus, and detecting any analyte present in the oral fluid by observing any detectable marker on the first test strip, and any color change on the second test strip.

Subject matter of the invention is also a method of preparing an apparatus for testing for the presence of drugs of abuse and alcohol in a sample, the method comprising:
providing a housing containing a sample collection pad partially extending from said housing; and
providing a single backing material supporting first and second test strips, said first test strip having components comprising a sample pad and an absorption pad in wicking communication for an immunological assay, said first test strip in wicking communication with said collection pad wherein said sample pad
and said absorption pad are each in wicking communication with said first test strip; and a second test strip having components for an enzymatic reaction, said second test strip being in wicking communication with said collection pad, the first and the second test strip are each associated with the collection pad, but not associated with one another.

In a preferred embodiment, said first strip further comprises a conjugate pad and a test membrane, and the method further comprises depositing a first label on said conjugate pad in such manner as to be freely movable therefrom in the presence of a fluid.

In a further preferred embodiment, the method further comprises depositing a control label on said conjugate pad in such manner as to be freely movable therefrom in the presence of a fluid.

In a preferred embodiment, the method further comprises immobilizing a plurality of analytes on said test membrane.

In a preferred embodiment of the method said second test strip further comprises a wicking strip and a reaction pad, and further comprising depositing a plurality of reagents for an enzymatic reaction on said reaction pad.

These and other advantages of the present invention will be apparent in light of the following figures and detailed description

### BRIEF DESCRIPTION OF THE DRAWINGS

FIG. 1 is a perspective view of the apparatus of the present invention, showing a top surface of the apparatus adapted to test for drugs of abuse;
FIG. 2 is a perspective view of the apparatus of the present invention, showing a bottom surface of the same apparatus adapted to test for alcohol;
FIG. 3A is a side view of components of the interior of the apparatus of the present invention showing components of first and second test strips for drugs of abuse and for alcohol;
FIG. 3B is a side view of components of the interior of an alternate embodiment of the apparatus of the present invention showing components of first and second test strips for drugs of abuse and for alcohol;
FIG. 4 is a schematic of a test of one embodiment of the present invention depicting sample added to the collection pad, marker-antibody conjugates at the conjugate pad, drug analytes bound at a test region, control analyte bound at a control region, and an absorbent pad;
FIG. 5 is a schematic of the test of FIG. 4 depicting drug analytes in the sample flowing to the conjugate pad and binding with marker-antibody conjugates; and
FIG. 6 is a schematic of the test of FIG. 4 after flow of sample has proceeded to the absorbent pad, depicting binding of some marker-antibody conjugates in the test region, and binding of control conjugate in the control region.

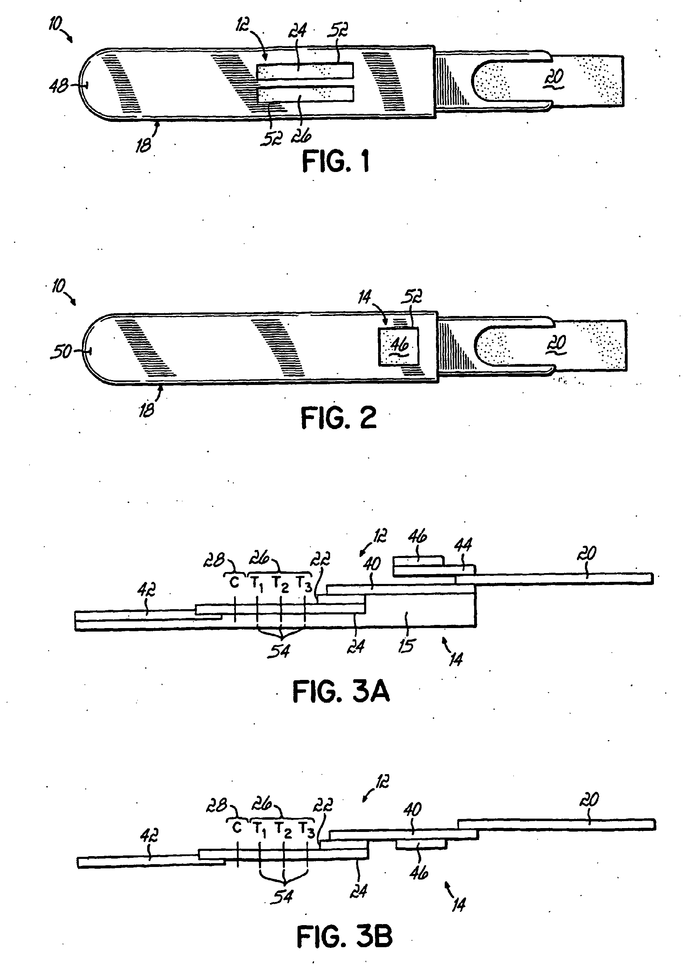

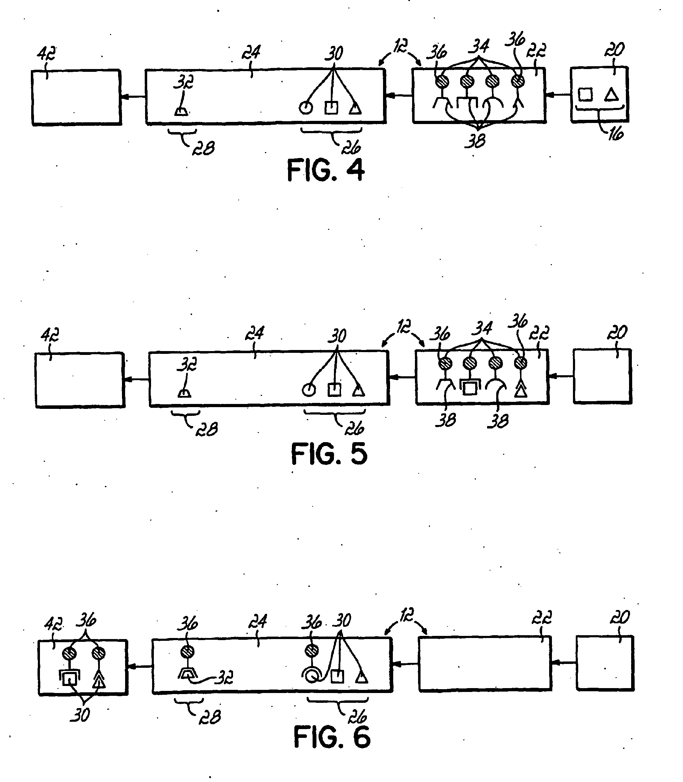

Figs. 4-6 are not drawn to scale to correspond to Figs. 1-3.

The accompanying drawings, which are incorporated in and constitute a part of the specification, illustrate embodiments of the invention and, together with a general description of the invention given above, and the detailed description of the embodiments given below, serve to explain the principles of the invention.

### DETAILED DESCRIPTION

A test apparatus and method whereby test strips for drugs of abuse and alcohol are provided in a single test apparatus. Such an apparatus and method reduces equipment, cost, and length of time of testing. It also provides a test that minimizes the invasiveness experienced by the test subject.

Referring now to the Figures, the invention is directed to an apparatus 10 that allows testing for the presence of both alcohol and non-alcohol drugs conventionally termed drugs of abuse, as will be further described, within a single apparatus. In general; the apparatus 10 of the present invention includes one or more first test strip 12, collectively called a "first test strip," although the apparatus is not limited to a single strip. The apparatus 10 further includes,a second test strip 14 for alcohol. More particularly, the first test strip 12 may include an immunoassay to screen for various drugs of abuse and the second test strip 14 may include a reactive pad including at least one enzyme reactive with alcohol. The present invention modifies both the physical and chemical character of these tests to achieve the goal of assaying both types of analytes within a single test device.

Although a combined test apparatus 10 as described herein can be used with many different types of samples 16, such as blood, urine, etc., in a particular embodiment, oral fluid is used as a sample 16. Since oral fluid is used instead of blood, urine, or another bodily fluid as the test sample 16, the apparatus 10 avoids the problems described above of intruding on privacy, invasiveness to the person, and can also avoid the problem of sample alteration. Further, due to their simple and rapid nature, the test strips 12,14 can be used on job sites, in schools, and customized for quick screening of illegal use of controlled materials.

More specifically, and referring to Figs. 1-3, the apparatus 10 of the present invention includes a housing 18 for containing components of the testing device, including reagents and material for an assay for drugs of abuse, reagents and material for an assay for alcohol, and including a collection pad 20 for collection of oral fluid. As used herein, the term "drugs of abuse" excludes alcohol but includes all drugs of abuse, since a separate test for alcohol is provided with the same device. In the illustrated embodiment, the collection pad 20 extends from the housing 18 such that this portion can be readily inserted into the mouth. The collection pad 20 includes an absorbent material that, when placed in the mouth of a subject, absorbs oral fluid. The oral fluid flows along the collection pad 20 via a wicking action to contact the sample pad of the drug of abuse first test strip 12 and the wicking pad of the alcohol second test strip 14. In the illustrated embodiment, a backing 15 is associated with the first test strip 12. This backing 15 thus supports the first test strip 12. It also provides indirect support (in the illustrated embodiment) to the second test strip 14. While the first and second test strips 12, 14 are each associated with the collection pad 20, they are not associated with one another in the illustrated embodiment. The first test strip 12 is used to test for drugs of abuse and the second test strip 14 is used to test for the presence of alcohol. As can be seen from the Figures, both the first test strip 12 and the second test strip 14 are disposed within the housing 18, in the illustrated embodiment.

Referring more particularly to Fig. 3A, the first test strip 12 (for drugs of abuse) of the apparatus 10 of the present invention includes at least a conjugate pad 22 associated with a test membrane 24. This first test strip 12 initiates at least one immunoassay that provides a visual qualitative result indicating the presence of one or more drugs and/or drug metabolites (referred to as "analytes") in the sample. In one embodiment, the test membrane 24 includes a test region 26, including one or more reaction zones 54, and a control region 28. The test region 26 includes drug analytes and protein conjugates (both represented by reference number 30) for particular drugs of abuse being tested. These drug analytes and protein conjugates 30 are immobilized on the membrane 24 in the test region 26. The control region 28 includes a control analyte 32 immobilized to the membrane 24. In a particular embodiment, this control analyte 32 is goat anti-rabbit antibody coated on or otherwise bound to the membrane 24 at the control region 28. The conjugate pad 22 includes marker-antibody conjugates 34 disposed thereon in such manner that they are freely movable therefrom. The term °marker-antibody conjugate" as used herein refers to a mobile labeled anti-analyte antibody. The apparatus 10 of the present invention further include a sample pad 40 and an absorption pad 42 associated with the first test strip 12. The first test strip 12 has components comprising a sample pad 40 and an absorption pad 42, wherein said sample pad 40 and said absorption pad 42 are each in wicking communication. In the illustrated embodiment, it can be seen that the sample pad 40 is in contact with both the collection pad 20 and the conjugate pad 22. The absorption pad 42 is located at the end of the apparatus 10 in the direction of sample flow and associated with the membrane 24 of the first test strip 12.

The second test strip 14 of the apparatus 10 of the present invention, in the illustrated embodiment, includes a wicking strip 44 in wicking association with a reaction pad 46. The wicking strip 44 in the illustrated embodiment, is associated with the collection pad 20. As a result, the test apparatus 10 of the present invention includes first and second sides 48, 50, each of the first and second sides 48, 50 including at least one window 52 for obtaining results of the tests of the apparatus 10. As can be seen in Fig. 1, the illustrated embodiment includes two windows 52 disposed over the test and control regions 26, 28 of the membrane 24 of the first test strip 12, and refeming to Fig. 2, the second side 50 of the apparatus 10 includes one window 52 disposed over the reaction pad 46 of the second test strip 14.

As described above, the apparatus 10 further includes test reagents on each of the first and second test strips 12, 14, which are used to detect the presence of any sought-after drug or alcohol analytes. More specifically, the conjugate pad 2 includes marker-antibody conjugates adapted to bind analyte through an analyte-specific antibody 38. The first test strip 12 further includes at least a first drug analyte and protein conjugate 30 bound at a test region 26 on the first test strip 12. If the sample 16 includes drug analytes, the analytes will be bound by the marker-antibody conjugate 34 and thus the marker-antibody conjugate 34 will not bind drug analyte and protein conjugate 30 immobilized at the test region 26. If there is no drug analyte in sample 16, then the marker-antibody conjugate will bind drug analyte and protein conjugate 30 immobilized in the test region 26. The marker-antibody conjugate 34 bound to drug analyte and protein conjugate at the test region 26 will create a detectable signal, indicating a negative test. This test will be described in greater detail later. Although a competitive immunoassay including marker that signals a negative test, as used in the apparatus 10 of the present invention, it will be recognized by those of skill in the art that the invention is not limited to this type of test, as other types of tests may be used. The invention is also not limited to the particular alcohol test described herein, as will be recognized by those of skill in the art.

The membrane 24 may be made of nitrocellulose or activated nylon. Immunoassay reagents are dried and immobilized on the membrane 24. In the illustrated embodiment, the first test strip 12 contains components for the immunoassay, being preformulated reagents deposited at multiple separate reaction zones 54 within the test region 26 of the membrane 24, one reaction zone 54 for each drug of abuse being tested. For example, the apparatus 10 of the present invention may include reaction zones 54, with each reaction zone 54 including one type of immobilized drug analyte and protein conjugate 30. Those drug analytes and protein conjugates 30 may be chosen from cocaine, d-methamphetamine, 11-nor-Δ9-tetrahydrocannabinol, d-amphetamine, opiates, and phencyclidine, etc. It will be recognized by those skilled in the art that these drug analytes and protein conjugates 30 are merely exemplary, and that one may immobilize an analyte of any substance one wishes to test in a particular reaction zone 54.

Regarding the drugs of abuse assay, pharmacokinetics of cocaine, opiates, amphetamine, methamphetamine, phencyclidine, and cannabinoids, show that these drugs are detectable in bodily fluids, such as oral fluid. Alcohol is also detectable in oral fluid. The apparatus 10 of the present invention integrates oral fluid collection and a lateral flow immunoassay screening test for drugs of abuse and an enzymatic assay for ethanol in a single integrated device.

The drugs of abuse test may be based on a competitive immunoassay procedure in which drug analytes and protein conjugates 30 immobilized on the membrane 24 of the first test strip 12 compete with drug or analytes, which may be present in oral fluid, for limited antibody binding sites 38 on the marker-antibody conjugate 34. In a particular embodiment, the detectable marker 36 is colloidal gold. However, those skilled in the art will recognize that any other detectable marker 36 may be used, including those detectable visually by the naked eye, those which fluoresce, or other non-visual methods of detection. During testing, oral fluid is collected at the collection pad 20 and migrates along the flow path of membrane 24: If no drug is present in the oral fluid, the marker-antibody conjugate 34 will bind to the drug analytes and protein conjugates 30 on the membrane 24 to form bands at specific reaction zones 54 in the test region 26. When colloidal gold is used as the marker, those bands are visible. Therefore, the presence of a colored band at a specific reaction zone 54 indicates a negative result for that specific drug. If any drug or drugs are present in the oral fluid, it competes with the immobilized drug analytes and protein conjugates 30 for limited antibody binding sites of the marker-antibody conjugate 34. When a sufficient amount of drug is present, the drug will saturate the antibodies, and the detectable marker-antibody conjugate 34 cannot bind to the drug analytes and protein conjugates 30 on the membrane 24. Therefore, the absence of a color band at the visually observable test region 26 indicates a positive result for that particular test.

A control is also provided for the first test strip 12 of the apparatus 10 of the present invention, to ensure that the test procedure has been performed properly. A band should always appear at the control region 28 on the membrane 24 regardless of the presence of drug or drug metabolite to indicate the device and components are operable.

More specifically, and referring to Figs. 4-6, the marker 36, being colloidal gold or other microparticles, such as colored latex particles, are sensitized with specific purified antibodies to the drugs of the test and a control, and are deposited on the conjugate pad 22 along the flow path of the sample 16. Colloidal gold may range in size from 10 nm to 60 nm. Microparticles, such as colored latex particles, may be about 400 nm. These marker-antibody conjugates are not immobilized on the conjugate pad 22, but rather, are freely movable therefrom. The particles of the detectable marker 36 may be any color. The detectable marker 36 is blocked with a protein buffer solution to prevent nonspecific aggregation.

The test is performed as follows. The sample 16 is applied onto the collection pad 20 and is wicked to the conjugate pad 22. In the illustrated embodiment, a sample pad 40 is disposed between collection pad 20 and conjugate pad 22 (Fig. 3). As the sample 16 is wicked through the conjugate pad 22, it mobilizes the marker-antibody conjugates 34 on the conjugate pad 22. The marker-antibody conjugates 34 then are wicked from the conjugate pad 22 to the membrane 24 by a wicking action (such as capillary action) and to and past the reaction zones 54 of the test region 26. In the absence of drug analyte(s) in the sample 16 that are specific for the drug analytes and protein conjugates 30 in the first test strip 12, the marker-antibody conjugates 34 bind to the specific drug analytes and protein conjugates 30 that are immobilized in the reaction zones 54 through immunocomplexation, forming definite, visible lines. This is a negative test if a line appears in a reaction zone 54 specific for a particular drug of abuse, the test is negative for that particular drug of abuse.

In the presence of specific analytes in the sample 16, the marker-antibody conjugate 34 in the conjugate pad 22 will bind to those analytes in the sample, and thus so not bind to the drug analytes and protein conjugates 30 that are immobilized in the reaction zones 54. Thus, the immune complex formation between the drug analytes and protein conjugates 30 in the reaction zones 54 and the marker-antibody conjugate 34 is prevented. In a positive test, therefore, lines will not form in the reaction zones 54.

At one end of the membrane 24 is the reference control region 28 with a different antigen antibody reaction. In particular, goat anti-rabbit antibody is immobilized on the membrane 24. Conjugate 34 form the conjugate pad including a detectable marker 36, always binds at the control region 28 during a viable test. The line created in the control region 28 indicates that the test is viable and serves as a reference control. If the test device has been properly stored, is correctly used, and is within the expiration time limit, the control line should always be present. Therefore, in the illustrated embodiment, for all negative tests, there will be visible lines. Thus, with multiple analytes being tested for, if all tests are negative, there will be lines visible in each of the reaction zones 54. If only certain tests are negative, there will be lines visible in some reaction zones 54 and absent in others. For positive tests for each analyte, there will be only one visible line, i.e., the reference control line. It will be further recognized by those skilled in the art that lines need not be used to indicate test and/or control results. Other symbols may be used, including but not limited to "+," "-," "POS," and "NEG."

Although the test device above is described as having a first test strip 12, such an embodiment is merely exemplary and it will be recognized that multiple immunochromatographic test strips may be present in the housing of the device of the present invention. As one example, the device may include two immunoassay test strips, with reaction and control zones, one strip being visible through a first window and the other strip being visible through a second window. As another example, the device may include two immunoassay test strips with both being visible through the same window. As another example, the device may include more than two test strips, with test and control zones visible through the same or separate windows.

The housing 18 of the apparatus 10 of the present invention not only includes the drugs of abuse test as described above, but also includes a test for the presence of alcohol. It is well established that the concentration of alcohol in oral fluid is comparable to that of alcohol in blood. The test for alcohol, in a particular embodiment of the present invention, is an enzymatic test. The enzymatic test for alcohol includes an enzymatic reaction pad 46, including alcohol oxidase and peroxidase. This reaction pad 46 is in wicking communication to a wicking membrane which is, in turn, in wicking communication to the collection pad 20. The reaction pad 46, on contact with solutions of alcohol, such as alcohol present in oral fluid, will rapidly turn different colors, in one embodiment from shades of green to blue, depending on the amount of alcohol present. The test is used by applying a sample, such as oral fluid, to the wicking membrane. The sample then moves via wicking action to the enzymatic reaction pad 46.

The reaction pad 46 contains a reagent system. The reagent system, as described briefly above and in more detail here, is made up of stabilized alcohol oxidase, a peroxidase, and a chromogen including color-changing oxygen acceptor. It may contain other materials as well. "Stabilized alcohol oxidase" can mean alcohol oxidase that retains at least 50% of its activity, in one embodiment, and at least 70% of its activity, in another embodiment, when stored in dried form at 56°C for fifteen days. The stabilized alcohol oxidase in its simplest form comprises alcohol oxidase in intimate admixture with an effective stabilizing concentration of stabilizing proteins.

The reagent system further includes a peroxidase (any material having peroxidative activity). This material promotes the reaction of the hydrogen peroxide, generated by the reaction of ethanol with oxygen, with the color-changing oxygen acceptor. While enzymatic plant peroxidases such as horseradish peroxidase or potato peroxidase may be employed, various other organic or inorganic peroxidases may be employed as well. These include organics such as some of the porphyrins, as well as inorganics such as ammonium or alkali metal iodides, alkali metal chromic sulfates, iron to ferrocyanide, ferrous chloride, and iron sulfacyanate, and the like, as known to one skilled in the art. Yeast peroxidase may also be used. The peroxidase promotes reaction of hydrogen peroxide with the color-changing oxygen acceptor.

In one embodiment, the reagent system includes tetramethylbenzidine (0.176 mg), alcohol oxidase (EC 1.1.3.1.3) (0.5 IU), peroxidase (EC 1.11.1.7) (30 IU), a buffer (0.747 mg), and stabilizing proteins (0.19 mg).

In one embodiment, the reaction pad 46 employs a, solid phase chemistry which uses the following highly specific enzyme reaction:

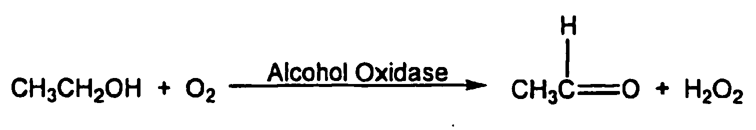

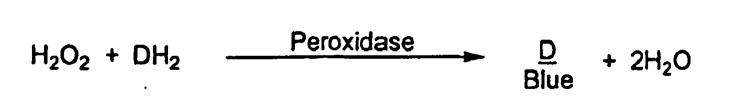

The alcohol test also includes a reaction inhibitor. This reaction inhibitor is used to control the timing of the result of the alcohol test by controlling the rate of the enzymatic reaction. The reaction inhibitor may be a quenching agent that consumes the hydrogen peroxide of the reagent system. In one embodiment, the quenching agent used as a reaction inhibitor is vitamin C.

The alcohol reaction pad 46 of the apparatus 10 of the present invention will react with methyl, ethyl, and allyl alcohols. The apparatus 10 may be formed such that the reaction of the alcohol test will not proceed in the presence of alcohols having 5 or more carbons, or with glycine, glycerol, or serine. The reaction will proceed in the presence of methyl, ethyl, and allyl alcohols. This property is a result of the specificity of the alcohol oxidase enzyme extracted from yeast.

One an alcohol test has been performed using the apparatus 10, the color of the reaction pad 46 may be compared to a color chart (such as may be located on the test package) in order to estimate the approximate blood alcohol concentration of the subject.

The integrity of the reaction pad 46 may be qualitatively versified using a test solution prepared by adding four drops of 80 proof distilled spirits to 8 oz. water. This solution may then be applied to the reaction pad. The solution should provide a color reaction equal to or higher (darker) than a color that would appear for 0.04% blood alcohol.

## Claims

1. An apparatus (10) for testing for multiple analytes, comprising:
- a housing (18) containing;
- a sample collection pad (20) partially extending from said housing (18), said sample collection pad in wicking communication with;
- a single backing material (15) supporting in a first test strip (12) components comprising a sample pad (40) and an absorption pad (42) in wicking communication for an immunological assay and in a second (14) test strip reagents for an enzymatic reaction;
- whereby a sample in the collection pad (20) communicates with both first (12) and second (14) test strips for both immunological and enzymatic detection of an analyte or analytes contained in the sample, wherein the first (12) and the second (14) test strip are each associated with the collection pad (20), but not associated with one another,
wherein said sample pad (40) and said absorption pad (42) are each in wicking communication with said first test strip.

2. The apparatus of claim 1, wherein said first test strip (12) and said second test strip (14) are disposed at least partially within said housing (18).

3. The apparatus of claim 1, wherein said sample collection pad (20) is adapted to receive a fluid sample, preferably wherein said fluid sample is oral fluid.

4. The apparatus of claim 1, wherein said first test strip (12) further comprises a conjugate pad (22) and a test membrane (24) in wicking communication with one another.

5. The apparatus of claim 4, further comprising a first label deposited on said conjugate pad (22) in such manner as to be freely movable therefrom in the presence of a fluid.

6. The apparatus of claim 5, wherein said first label comprises an analyte antibody conjugated to a detectable marker.

7. The apparatus of claim 6, wherein said first label includes a plurality of antibodies to a plurality of analytes, each of said plurality of antibodies being conjugated to a detectable marker.

8. The apparatus of claim 7, wherein said detectable marker is selected from the group consisting of colloidal gold, and colored latex particles.

9. The apparatus of claim 4, further comprising a control label deposited on said conjugate pad (22), said control label being freely movable therefrom in the presence of a fluid.

10. The apparatus of claim 4, further comprising a plurality of analytes immobilized on said test membrane (24), preferably wherein said plurality of analytes is selected from the group consisting of analytes of cocaine, d-methamphetamine, 11-nor-[Delta]9-tetrahydrocannabinol, d- amphetamine, opiates, and phencyclidine.

11. The apparatus of claim 10, wherein said test membrane (24) includes a plurality of reaction zones, wherein each of said plurality of reaction zones includes one of said plurality of analytes immobilized therein, each of said plurality of subsets in including multiple analytes of a single type.

12. The apparatus of claim 11, further comprising a plurality of labels, each of said plurality of labels including an analyte antibody conjugated to a detectable marker, each of said antibodies being specific to one of said plurality of analytes, said plurality of labels being deposited on said conjugate pad and freely movable therefrom in the presence of fluid, such that when said antibodies bind to said plurality of analytes in said reaction zones in a predetermined concentration, said detectable marker is visible to the human eye.

13. The apparatus of claim 1, wherein said second test strip (14) further comprises a wicking strip (44) and a reaction pad (46).

14. The apparatus of claim 13, wherein said reagents are disposed on said reaction pad (46), preferably wherein said reagents are selected from the group consisting of tetramethylbenzidine, alcohol oxidase, peroxidase, buffers, and stabilizing proteins.

15. The apparatus of claim 14, wherein said reagents produce an enzymatic reaction in the presence of alcohol.

16. The apparatus of claim 15, wherein said reaction pad (46) exhibits a first color in the absence of alcohol and exhibits a second color in the presence of alcohol.

17. The apparatus of claim 1 , wherein said housing (18) further comprises at least one window (52) through which a reaction area of a nitrocellulose membrane can be observed.

18. A method for testing for both drugs of abuse and alcohol, the method comprising: providing an apparatus (10) according to at least one of claims 1 to 17, providing an oral fluid sample to the collection pad (20) thereby initiating said immunological assay and said enzymatic reaction; and detecting results of said immunological assay and said enzymatic reaction.

19. A method of preparing an apparatus (10) for testing for the presence of drugs of abuse and alcohol in a sample, the method comprising: providing a housing (18) containing a sample collection pad (20) partially extending from said housing; and providing a single backing material (15) supporting first (12) and second (14) test strips, said first test strip (12) having components comprising a sample pad (40) and an absorption pad in wicking communication for an immunological assay, said first test strip (12) in wicking communication with said collection pad(20); and a second test strip (14) having components for an enzymatic reaction, said second test strip (14) being in wicking communication with said collection pad (20), wherein the first (12) and the second test strip (14) are each associated with the collection pad (20), but not associated with one another,
wherein said sample pad (40) and said absorption pad (42) are each in wicking communication with said first test strip.

## Patentansprüche

1. Vorrichtung (10) zum Testen auf multiple Analyten, umfassend:
- ein Gehäuse (18), enthaltend:
- ein Probensammelfeld (20), das sich partiell von dem Gehäuse (18) weg erstreckt, wobei sich das Probensammelfeld in Kapillarverbindung befindet mit:
- einem einzigen Trägermaterial (15), das in einem ersten Teststreifen (12) Komponenten, die ein Probenfeld (40) und ein Absorptionsfeld (42) umfassen, in Kapillarverbindung für einen immunologischen Assay und in einem zweiten Teststreifen (14) Reagentien für eine enzymatische Reaktion trägt;
- wodurch eine Probe in dem Sammelfeld (20) sowohl mit dem ersten (12) als auch mit dem zweiten (14) Teststreifen in Verbindung steht für sowohl einen immunologischen als auch einen enzymatischen Nachweis eines oder mehrerer Analyten, die in der Probe enthalten sind, wobei der erste (12) und der zweite (14) Teststreifen jeweils mit dem Sammelfeld (20) assoziiert sind, aber nicht miteinander assoziiert sind;
wobei sich das Probenfeld (40) und das Absorptionsfeld (42) jeweils in Kapillarverbindung mit dem ersten Teststreifen befinden.

2. Vorrichtung gemäß Anspruch 1, wobei sich der erste Teststreifen (12) und der zweite Teststreifen (14) wenigstens teilweise innerhalb des Gehäuses (18) befinden.

3. Vorrichtung gemäß Anspruch 1, wobei das Probensammelfeld (20) eine flüssige Probe aufnehmen kann, wobei es sich bei der flüssigen Probe vorzugsweise um Mundflüssigkeit handelt.

4. Vorrichtung gemäß Anspruch 1, wobei der erste Teststreifen (12) weiterhin ein Konjugatfeld (22) und eine Testmembran (24) in Kapillarverbindung miteinander umfasst.

5. Vorrichtung gemäß Anspruch 4, die weiterhin eine erste Markierung umfasst, die auf dem Konjugatfeld (22) in einer solchen Weise abgelegt ist, dass sie sich in Gegenwart einer Flüssigkeit frei von dem Konjugatfeld weg bewegen kann.

6. Vorrichtung gemäß Anspruch 5, wobei die erste Markierung einen Analytenantikörper umfasst, der mit einem nachweisbaren Marker konjugiert ist.

7. Vorrichtung gemäß Anspruch 6, wobei die erste Markierung eine Vielzahl von Antikörpern gegen eine Vielzahl von Analyten umfasst, wobei jeder der Vielzahl von Antikörpern mit einem nachweisbaren Marker konjugiert ist.

8. Vorrichtung gemäß Anspruch 7, wobei der nachweisbare Marker aus der Gruppe ausgewählt ist, die aus kolloidalem Gold und farbigen Latexteilchen besteht.

9. Vorrichtung gemäß Anspruch 4, die weiterhin eine Kontrollmarkierung umfasst, die auf dem Konjugatfeld (22) abgelegt ist, wobei sich die Kontrollmarkierung in Gegenwart einer Flüssigkeit frei von dem Konjugatfeld weg bewegen kann.

10. Vorrichtung gemäß Anspruch 4, die weiterhin eine Vielzahl von Analyten umfasst, die auf der Testmembran (24) immobilisiert sind, wobei die Vielzahl von Analyten vorzugsweise aus der Gruppe ausgewählt ist, die aus Analyten von Kokain, d-Methamphetamin, 11-Nor-Δ9-tetrahydrocanna-binol, d-Amphetamin, Opiaten und Phencyclidin besteht.

11. Vorrichtung gemäß Anspruch 10, wobei die Testmembran (24) eine Vielzahl von Reaktionszonen umfasst, wobei in jeder der Vielzahl von Reaktionszonen einer der Vielzahl von Analyten immobilisiert ist, wobei jede der Vielzahl von Untergruppen multiple Analyten eines einzigen Typs umfasst.

12. Vorrichtung gemäß Anspruch 11, die weiterhin eine Vielzahl von Markierungen umfasst, wobei jede der Vielzahl von Markierungen einen Analytenantikörper umfasst, der mit einem nachweisbaren Marker konjugiert ist, wobei jeder der Antikörper spezifisch für einen der Vielzahl von Analyten ist, wobei die Vielzahl von Markierungen auf dem Konjugatfeld abgelegt sind und sich in Gegenwart einer Flüssigkeit frei von dem Konjugatfeld weg bewegen können, so dass der nachweisbare Marker für das menschliche Auge sichtbar ist, wenn die Antikörper in einer vorbestimmten Konzentration an die Vielzahl von Analyten in den Reaktionszonen binden.

13. Vorrichtung gemäß Anspruch 1, wobei der zweite Teststreifen (14) weiterhin einen Kapillarstreifen (44) und ein Reaktionsfeld (46) umfasst.

14. Vorrichtung gemäß Anspruch 13, wobei sich die Reagentien auf dem Reaktionsfeld (46) befinden, wobei die Reagentien vorzugsweise aus der Gruppe ausgewählt sind, die aus Tetramethylbenzidin, Alkohol-Oxidase, Peroxidase, Puffern und stabilisierenden Proteinen besteht.

15. Vorrichtung gemäß Anspruch 14, wobei die Reagentien in Gegenwart von Alkohol eine enzymatische Reaktion erzeugen.

16. Vorrichtung gemäß Anspruch 15, wobei das Reaktionsfeld (46) in Abwesenheit von Alkohol eine erste Farbe aufweist und in Gegenwart von Alkohol eine zweite Farbe aufweist.

17. Vorrichtung gemäß Anspruch 1, wobei das Gehäuse (18) weiterhin wenigstens ein Fenster (52) umfasst, durch das ein Reaktionsbereich einer Nitrocellulosemembran beobachtet werden kann.

18. Verfahren zum Testen sowohl auf Drogen als auch auf Alkohol, wobei das Verfahren Folgendes umfasst: Bereitstellen einer Vorrichtung (10) gemäß wenigstens einem der Ansprüche 1 bis 17, Bereitstellen einer Mundflüssigkeitsprobe auf dem Sammelfeld (20), wodurch der immunologische Assay und die enzymatische Reaktion eingeleitet werden, und Nachweisen der Ergebnisse des immunologischen Assays und der enzymatischen Reaktion.

19. Verfahren zur Herstellung einer Vorrichtung (10) zum Testen auf die Anwesenheit von Drogen und Alkohol in einer Probe, wobei das Verfahren Folgendes umfasst: Bereitstellen eines Gehäuse (18), das ein Probensammelfeld (20) enthält, das sich partiell von dem Gehäuse weg erstreckt; und Bereitstellen eines einzigen Trägermaterials (15), das einen ersten (12) und einen zweiten Teststreifen (14) trägt, wobei der erste Teststreifen (12) Komponenten aufweist, die ein Probenfeld (40) und ein Absorptionsfeld in Kapillarverbindung mit dem Sammelfeld (20) umfassen, und der zweite Teststreifen (14) Komponenten für eine enzymatische Reaktion aufweist, wobei sich der zweite Teststreifen (14) in Kapillarverbindung mit dem Sammelfeld (20) befindet, wobei der erste (12) und der zweite Teststreifen (14) jeweils mit dem Sammelfeld assoziiert sind, aber nicht miteinander assoziiert sind;
wobei sich das Probenfeld (40) und das Absorptionsfeld (42) jeweils in Kapillarverbindung mit dem ersten Teststreifen befinden.

## Revendications

1. Appareil (10) pour mettre à l'essai de multiples analytes, comprenant :
- un logement (18) contenant
- une plaquette de collecte d'échantillon (20) s'étendant partiellement à partir dudit logement (18), ladite plaquette de collecte d'échantillon étant en communication par capillarité avec
- un matériau de renfort unique (15) supportant, dans une première bandelette d'essai (12), des composants comprenant une plaquette d'échantillon (40) et une plaquette d'absorption (42) en communication par capillarité pour un dosage immunologique et, dans une seconde bandelette d'essai (14), des réactifs pour une réaction enzymatique ;
- moyennant quoi un échantillon dans la plaquette de collecte (20) communique avec les première (12) et seconde (14) bandelettes d'essai à la fois pour une détection immunologique et enzymatique d'un analyte ou de plusieurs analytes contenus dans l'échantillon, où les première (12) et seconde (14) bandelettes d'essai sont chacune associées à la plaquette de collecte (20), mais pas associées l'une à l'autre,
où ladite plaquette d'échantillon (40) et ladite plaquette d'absorption (42) sont chacune en communication par capillarité avec ladite première bandelette d'essai.

2. Appareil selon la revendication 1, dans lequel ladite première bandelette d'essai (12) et ladite seconde bandelette d'essai (14) sont disposées au moins partiellement au sein dudit logement (18).

3. Appareil selon la revendication 1, dans lequel ladite plaquette de collecte d'échantillon (20) est adaptée pour recevoir un échantillon de fluide, de préférence dans lequel ledit échantillon de fluide est un fluide oral.

4. Appareil selon la revendication 1, dans lequel ladite première bandelette d'essai (12) comprend en outre une plaquette de conjugué (22) et une membrane d'essai (24) en communication par capillarité l'une avec l'autre.

5. Appareil selon la revendication 4, comprenant en outre un premier traceur déposé sur ladite plaquette de conjugué (22) de sorte à être librement amovible de celle-ci en présence d'un fluide.

6. Appareil selon la revendication 5, dans lequel ledit premier traceur comprend un anticorps d'analyte conjugué à un marqueur détectable.

7. Appareil selon la revendication 6, dans lequel ledit premier traceur inclut une pluralité d'anticorps à une pluralité d'analytes, chaque anticorps de ladite pluralité d'anticorps étant conjugué à un marqueur détectable.

8. Appareil selon la revendication 7, dans lequel ledit marqueur détectable est choisi dans le groupe constitué par l'or colloïdal et les particules de latex colorées.

9. Appareil selon la revendication 4, comprenant en outre un traceur témoin déposé sur ladite plaquette de conjugué (22), ledit traceur témoin étant librement mobile de celle-ci en présence d'un fluide.

10. Appareil selon la revendication 4, comprenant en outre une pluralité d'analytes immobilisés sur ladite membrane d'essai (24), de préférence dans lequel ladite pluralité d'analytes est choisie dans le groupe constitué par les analytes de cocaïne, d-méthamphétamine, 11-nor-[Delta]9-tétrahydrocannabinol, d-amphétamine, opiacés et phencyclidine.

11. Appareil selon la revendication 10, dans lequel ladite membrane d'essai (24) inclut une pluralité de zones de réaction, chaque zone de ladite pluralité de zones de réaction incluant l'un des analytes de ladite pluralité d'analytes immobilisés dans celle-ci, chaque sous-ensemble de ladite pluralité de sous-ensembles incluant de multiples analytes d'un seul type.

12. Appareil selon la revendication 11, comprenant en outre une pluralité de traceurs, chaque traceur de ladite pluralité de traceurs incluant un anticorps d'analyte conjugué à un marqueur détectable, chacun desdits anticorps étant spécifique d'un analyte de ladite pluralité d'analytes, ladite pluralité de traceurs étant déposée sur ladite plaquette de conjugué et librement amovible de celle-ci en présence d'un fluide, de sorte que lorsque lesdits anticorps se lient à ladite pluralité d'analytes dans lesdites zones de réaction à une concentration prédéterminée, ledit marqueur détectable est visible à l'oeil humain.

13. Appareil selon la revendication 1, dans lequel ladite seconde bandelette d'essai (14) comprend en outre une mèche capillaire (44) et une plaquette de réaction (46).

14. Appareil selon la revendication 13, dans lequel lesdits réactifs sont disposés sur ladite plaquette de réaction (46), de préférence dans lequel lesdits réactifs sont choisis dans le groupe constitué par la tétraméthylbenzidine, l'alcool oxydase, la peroxydase, des tampons, et des protéines stabilisantes.

15. Appareil selon la revendication 14, dans lequel lesdits réactifs produisent une réaction enzymatique en présence d'alcool.

16. Appareil selon la revendication 15, dans lequel ladite plaquette de réaction (46) présente une première couleur en l'absence d'alcool et présente une seconde couleur en présence d'alcool.

17. Appareil selon la revendication 1, dans lequel ledit logement (18) comprend en outre au moins une fenêtre (52) à travers laquelle une zone de réaction d'une membrane de nitrocellulose peut être observée.

18. Procédé de mise à l'essai à la fois de drogues et d'alcool, le procédé comprenant : la fourniture d'un appareil (10) selon au moins l'une des revendications 1 à 17, la fourniture d'un échantillon de fluide oral sur la plaquette de collecte (20), initiant ainsi ledit dosage immunologique et ladite réaction enzymatique ; et la détection des résultats dudit dosage immunologique et de ladite réaction enzymatique.

19. Procédé de préparation d'un appareil (10) pour mettre à l'essai la présence de drogues et d'alcool dans un échantillon, le procédé comprenant : la fourniture d'un logement (18) contenant une plaquette de collecte d'échantillon (20) s'étendant partiellement à partir dudit logement ; et la fourniture d'un matériau de renfort unique (15) supportant des première (12) et seconde (14) bandelettes d'essai, ladite première bandelette d'essai (12) ayant des composés comprenant une plaquette d'échantillon (40) et une plaquette d'absorption en communication par capillarité pour un dosage immunologique, ladite première bandelette d'essai (12) étant en communication par capillarité avec ladite plaquette de collecte (20) ; et une seconde bandelette d'essai (14) ayant des composants pour une réaction enzymatique, ladite seconde bandelette d'essai (14) étant en communication par capillarité avec ladite plaquette de collecte (20), où lesdites première (12) et seconde (14) bandelettes d'essai sont chacune associées à la plaquette de collecte (20), mais non associées l'une à l'autre,
où ladite plaquette d'échantillon (40) et ladite plaquette d'absorption (42) sont chacune en communication par capillarité avec ladite première bandelette d'essai.
